# EUROPEAN PATENT APPLICATION

(11) **EP 4 850 587 A1**
(43) Date of publication of application: **09.12.2026**
(21) Application number: 25751284.8
(22) Date of filing: 23.01.2025
(51) Int. Cl.: A61B 1/012, A61B 1/04, A61B 1/06, A61B 1/018

(54) **ENDOSCOPE AND DISTAL END THEREOF**

(30) Priority: 05.02.2024 CN 202410173289
(71) Applicant: Sonoscape Medical Corp., Shenzhen, Guangdong 518057 (CN)
(72) Inventor: GONG, Pengcheng, Shenzhen, Guangdong 518057 (CN); JING, Feng, Shenzhen, Guangdong 518057 (CN); PI, Hao, Shenzhen, Guangdong 518057 (CN)
(74) Representative: IPAZ
(86) International application number: PCT/CN2025/074245
(87) International publication number: WO 2025/167682

(57) **Abstract**

According to embodiments of the present disclosure, an endoscope and a distal end portion for endoscope are provided. The distal end portion comprises a distal end base and a raising base. The distal end portion comprises a distal end base and a raising base. The distal end base has an accommodation chamber defined therein. The accommodation chamber communicates with an instrument channel of the endoscope and has an exit window for a medical instrument to protrude therethrough. An abutment portion is provided at an edge of the exit window. The raising base is rotatably disposed in the accommodation chamber and configured to adjust a direction in which the medical instrument protrudes from the exit window. The raising base comprises a rotating shaft end and a distal end. The raising base comprises a first guide surface extending from the distal end toward the rotating shaft end. The first guide surface is configured to support the medical instrument and guide the medical instrument along a predetermined extension path or a predetermined retraction path. The first guide surface comprises an abutment position corresponding to the abutment portion. The abutment position is configured to cooperate with the abutment portion to clamp the medical instrument supported on the first guide surface when the raising base is lifted to a predetermined angle. The raising base further comprises a positioning groove at the distal end. At least a portion of the positioning groove is formed in the first guide surface. The positioning groove is configured to maintain the medical instrument in the extension path or the retraction path in position, and the abutment position is located outside the positioning groove. Based on the above solution, the present disclosure can improve the convenience of medical instrument replacement.

## Description

### TECHNICAL FIELD

The present disclosure generally relates to the technical field of endoscopes, and specifically to an endoscope and a distal end portion for endoscope.

### BACKGROUND

Endoscopic imaging, a non-invasive imaging method that effectively extends human vision, is widely applied in image diagnosis and image-guided therapy across multiple fields, including the digestive tract, cardiovascular and cerebrovascular systems, urinary system and respiratory system, thereby greatly improves the disease examination accuracy.

An endoscope comprises an elongated insertion unit configured to enter a human body, the insertion unit generally defining an instrument channel. During diagnosis or treatment, a medical instrument can be advanced through the instrument channel to a distal end of the endoscope. In order to enable the medical instrument to reach a suitable position after extending out of the instrument channel, a guide wire may be used to guide the medical instrument. The guide wire may be delivered to a target position by means of a cannula. In a typical procedure, the cannula is first advanced through the instrument channel to the target position in the human body; the guide wire is then introduced into a lumen of the cannula, thereby also reaching the target position; subsequently, the cannula is withdrawn, and another medical instrument, such as a papillotome, can be advanced along the guide wire to the target position. However, during withdrawal of the cannula or advancement of the papillotome, friction between the guide wire and the cannula or between the guide wire and the papillotome tends to displace the guide wire from its intended position. To counteract this, during withdrawal of the cannula or insertion of the papillotome by a doctor, a nurse is usually required to manually hold the guide wire, applying a counterforce opposite to the friction direction while simultaneously stabilizing the guide wire to prevent lateral shaking, so as to maintain its insertion position. Such manual coordination is not only time-consuming and labor-intensive.

### SUMMARY

In order to at least partially solve the problems existing in the prior art, according to some embodiments of the present disclosure, a distal end portion of an endoscope is provided. The distal end portion comprises a distal end base and a raising base. The distal end base has an accommodation chamber defined therein. The accommodation chamber communicates with an instrument channel of the endoscope and has an exit window for a medical instrument to protrude therethrough. An abutment portion is provided at an edge of the exit window. The raising base is rotatably disposed in the accommodation chamber and configured to adjust a direction in which the medical instrument protrudes from the exit window. The raising base comprises a rotating shaft end and a distal end. The raising base comprises a first guide surface extending from the distal end toward the rotating shaft end. The first guide surface is configured to support the medical instrument and guide the medical instrument along a predetermined extension path or a predetermined retraction path. The first guide surface comprises an abutment position corresponding to the abutment portion. The abutment position is configured to cooperate with the abutment portion to clamp the medical instrument supported on the first guide surface when the raising base is lifted to a predetermined angle. The raising base further comprises a positioning groove at the distal end. At least a portion of the positioning groove is formed in the first guide surface. The positioning groove is configured to maintain the medical instrument in the extension path or the retraction path in position, and the abutment position is located outside the positioning groove.

In the distal end portion of the present disclosure, the medical instrument can be mechanically locked by being clamped between the abutment portion and the first guide surface, and is thereby maintained in position. The positioning groove may be configured to maintain the medical instrument located in the extension path or the retraction path in position. When the medical instrument is received in the positioning groove, relatively large friction can exist between the medical instrument and the positioning groove, such that the medical instrument is resistant to left-right shaking and/or forward-backward movement. Specifically, during replacement of the medical instrument under the guidance of the guide wire, for example, during retraction of the cannula along the guide wire in the instrument channel and during advancement of the papillotome along the guide wire in the instrument channel, the abutment portion and the first guide surface can clamp the guide wire, thereby mechanically locking the guide wire in position. However, during the process in which the distal tip of the cannula is retracted from the positioning groove to the abutment position (i.e., the mechanical locking position of the guide wire), or the process in which the distal tip of the papillotome is advanced from the abutment position (i.e., the mechanical locking position of the guide wire) to the positioning groove, although the cannula or the papillotome is sleeved over the guide wire so that the abutment portion cannot, together with the first guide surface, mechanically lock the guide wire, the portion of the guide wire exposed beyond the cannula or the papillotome is received in the positioning groove, so that the positioning groove can form a radial constraint on the guide wire, thereby preventing lateral shifting of the guide wire. In addition, relatively large friction between the positioning groove and the guide wire resists axial shifting of the guide wire. That is, when the distal tip of the medical instrument, such as a cannula or a papillotome, guided by the guide wire moves between the positioning groove and the abutment position, the position of the guide wire can be maintained by the friction between the positioning groove and the guide wire. When the distal tip of the medical instrument, such as a cannula or a papillotome, guided by the guide wire moves between the abutment position and the instrument channel inlet, the guide wire can be maintained not only by friction between the positioning groove and the guide wire, but also by mechanical clamping of the abutment portion and the first guide surface on the guide wire. Thus, the raising base of the present disclosure has a mechanical locking state and a semi-locking state. When replacing the medical instrument under the guidance of the guide wire, the position of the guide wire can be stably maintained, whether during replacement of the medical instrument on the raising base or during movement of the medical instrument in the instrument channel. An operator can use such an endoscope more simply and conveniently. In addition, in the present disclosure, since the abutment position for mechanically locking the medical instrument is on the first guide surface and outside the positioning groove, the surface where the abutment position is located has a relatively regular shape. As a result, the force on the guide wire can be more uniform, stress concentration can be reduced, and the medical instrument is less likely to be damaged. This avoids replacement of the medical instrument due to damage during use of the endoscope, thereby improving stability of the endoscope during use and enhancing the user experience.

Exemplarily, a positioning groove outlet is defined at the distal end of the raising base, and the positioning groove is formed by extending from the positioning groove outlet toward the rotating shaft end along a predetermined route. The medical instrument comprises a first-specification instrument which has a diameter less than or equal to a predetermined value such that at least half of the first-specification instrument is receivable in the positioning groove outlet.

Exemplarily, when an instrument supported on the first guide surface is the first-specification instrument and the raising base is lifted to the predetermined angle, in a cross section of the first-specification instrument, at least half of the positioning groove outlet is received in the positioning groove outlet.

Exemplarily, a cross section of the positioning groove outlet is V-shaped, and the cross section is perpendicular to the predetermined route.

Exemplarily, an opening of the positioning groove is tapered from the distal end toward the rotating shaft end along the predetermined route.

Exemplarily, the first guide surface extends along a longitudinal axis of the raising base, and the predetermined route intersects with the longitudinal axis of the raising base.

Exemplarily, the abutment position is spaced apart from the positioning groove with a non-zero spacing.

Exemplarily, a circle internally tangent to both the predetermined route and a central axis of the first guide surface, and passing through the positioning groove outlet and the abutment position, has a diameter greater than a minimum bending diameter of the first-specification instrument.

Exemplarily, the distal end portion further comprises an optical imaging assembly located on a same side of the distal end base as the exit window. The predetermined route is inclined toward a center of a field of view of the optical imaging assembly.

According to some embodiments of the present disclosure, an endoscope is provided. The endoscope comprises any one of the distal end portions as described above.

A series of concepts in simplified form are introduced in the Summary, which will be described in further detail in the Detailed Description section. This Summary section is not intended to attempt to define the key features and essential technical features of the claimed technical solution, nor is it intended to attempt to determine the scope of protection of the claimed technical solution.

Features and advantages of the present disclosure are described in detail below with reference to the accompanying drawings.

### BRIEF DESCRIPTION OF THE DRAWINGS

The following accompanying drawings of the present disclosure are used here as a part of the present disclosure for understanding the present disclosure. The embodiments and descriptions of the present disclosure are illustrated in the accompanying drawings to explain the principle of the present disclosure. In drawings:
Fig. 1 is a schematic view of an endoscope according to an exemplary embodiment of the present disclosure;
Fig. 2 is a perspective view of the endoscope partially showing the distal end portion, according to an exemplary embodiment of the present disclosure;
Fig. 3 is a perspective view of the raising base and adjacent components thereof in the endoscope shown in Fig. 2;
Fig. 4 is an exploded view of the raising base and the adjacent components thereof shown in Fig. 3;
Fig. 5 is a perspective view of the endoscope partially showing the distal end portion, according to an exemplary embodiment of the present disclosure, wherein a medical instrument is supported on the raising base, and the raising base is in an initial state;
Fig. 6 is a perspective view of the endoscope partially showing the distal end portion, according to an exemplary embodiment of the present disclosure, wherein a medical instrument is supported on the raising base, and the raising base is in a semi-locking state;
Fig. 7 is a cross-sectional view of the distal end portion of the endoscope shown in Fig. 6;
Fig. 8 is a perspective view of the endoscope partially showing the distal end portion, according to an exemplary embodiment of the present disclosure, wherein a medical instrument is supported on the raising base, and the raising base is in a mechanical locking state;
Fig. 9 is a cross-sectional view of the distal end portion of the endoscope shown in Fig. 8;
Fig. 10 is a perspective view of the raising base, observed in a direction parallel to the exit window, according to an exemplary embodiment of the present disclosure, wherein a medical instrument is supported on the raising base, and the raising base is in a mechanical locking state;
Fig. 11 is a perspective view of the endoscope partially showing the distal end portion, according to an exemplary embodiment of the present disclosure, wherein a medical instrument is supported on the raising base, and the raising base is in a mechanical locking state;
Fig. 12 is a partial enlarged view of the distal end portion of the endoscope shown in Fig. 11.

The above accompanying drawings include the following reference signs:
10, insertion unit; 100, distal end portion; 110, distal end base; 111, exit window; 112, abutment portion; 1121, abutment surface; 113, instrument channel outlet; 120, raising base; 121, rotating shaft end; 122, distal end; 123, first guide surface; 124, positioning groove; 1241, positioning groove outlet; 125, second guide surface; 126, rotating portion; 127, abutment position; 130, optical imaging assembly; 131, illumination window; 132, camera; 140, steel wire; 150, pin; 160, rotating arm; 170, distal end cap; 20, operation unit; 30, universal cable; 40, light guide unit; 50, first-specification instrument.

### DETAILED DESCRIPTION

In the following description, numerous details are provided to enable a thorough understanding of the present disclosure. However, a person skilled in the art may understand that the following description only exemplarily shows the preferred embodiments of the present disclosure, and the present disclosure may be implemented without one or more such details. In addition, in order to avoid confusion with the present disclosure, some technical features known in the art have not been described in detail.

According to an aspect of the present disclosure, a distal end portion of an endoscope is provided. Therefore, according to another aspect of the present disclosure, an endoscope is provided. The endoscope may include any one of the distal end portions to be described below. The endoscope and the distal end portion thereof provided by the present disclosure are described in detail hereinafter with reference to the accompanying drawings.

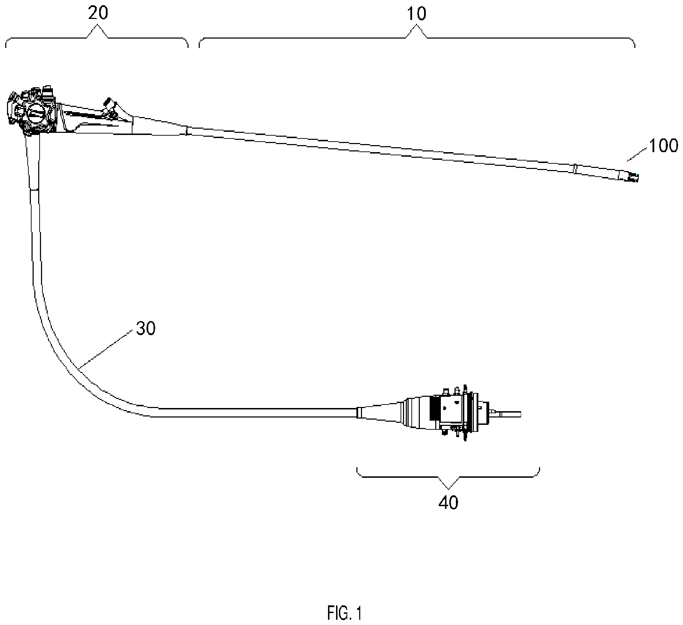

As shown in Fig. 1, the endoscope may include an insertion unit 10, an operation unit 20, a universal cable 30 and a light guide unit 40 connected in sequence.

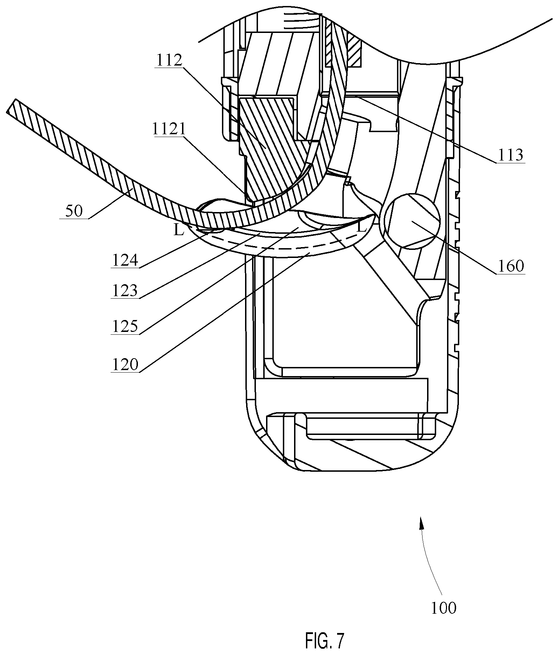

The insertion unit 10 generally may include an instrument channel. One end of the instrument channel extends to the operation unit 20 and forms an instrument channel inlet at the operation unit 20. The other end of the instrument channel extends to the distal end portion 100 of the insertion unit 10 to be described below, and forms an instrument channel outlet 113 at the distal end portion 100 (as shown in Fig. 7). In this way, a medical instrument may be inserted through the instrument channel inlet at the operation unit 20, and protrude out of the instrument channel outlet 113 at the distal end portion 100. An operator can manipulate the medical instrument from the operation unit 20 side, and perform auxiliary diagnosis or treatment using a distal end of the medical instrument protruding from the distal end portion 100. The distal end of the medical instrument protruding from the distal end portion 100 may be supported by a raising base 120 to be described below. The medical instrument may take any suitable form. For example, in endoscopic retrograde cholangiopancreatography, the medical instrument may include, but is not limited to, a cannula, a guide wire, a papillotome and the like.

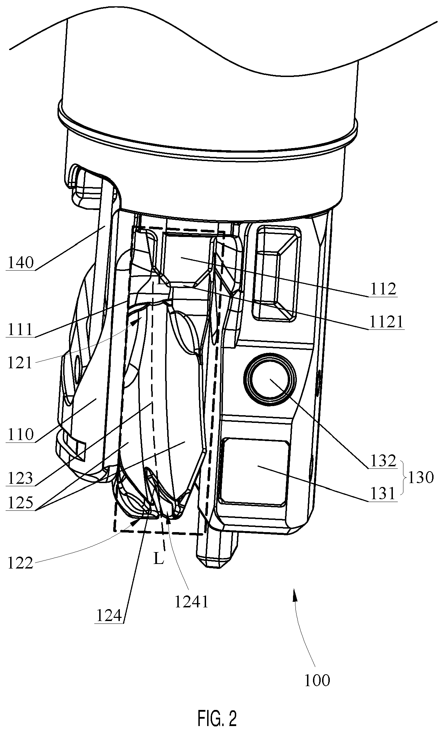

The insertion unit 10 may include the distal end portion 100. The distal end portion 100 may accommodate an assembly for endoscope imaging. For example, as shown in Fig. 2, an optical imaging assembly 130 may be accommodated in the distal end portion 100. The optical imaging assembly 130 may include an illumination window 131 and a camera 132. Generally, the distal end portion 100 is rigid.

A medical system may include a light source device and a host. The light guide unit 40 may be indirectly connected to the host by connecting to the light source device. Alternatively, the light guide unit 40 may also be directly connected to both the light source device and the host. Illuminating light generated by the light source device may be transmitted to the illumination window 131 of the distal end portion 100 via a light guide medium extending through the light guide unit 40, the universal cable 30, the operation unit 20 and the insertion unit 10, and then exit the illumination window 131 to irradiate a subject. The camera 132 of the distal end portion 100 can collect light returned from the subject, generate an optical image signal of the subject and transmit the signal to the light guide unit 40. The light guide unit 40 further transmits the signal to the host for image processing, to obtain an optical image of the subject.

It should be appreciated that the embodiment shown in Fig. 1 is described with reference to an optical endoscope. In unillustrated embodiments, the endoscope of the present disclosure may have various other applications. For example, the endoscope of the present disclosure may also be an ultrasonic endoscope. In such embodiments, the endoscope may further include an ultrasonic probe and an ultrasonic connector, and the medical system may correspondingly include an ultrasonic host.

The distal end portion of the embodiment of the present disclosure is described in detail below with reference to the accompanying drawings. For convenience of description, a "distal end" as used herein refers to an end of the endoscope closer to the subject when an operator uses the endoscope; a "proximal end" as used herein refers to an end of the endoscope closer to the operator during use.

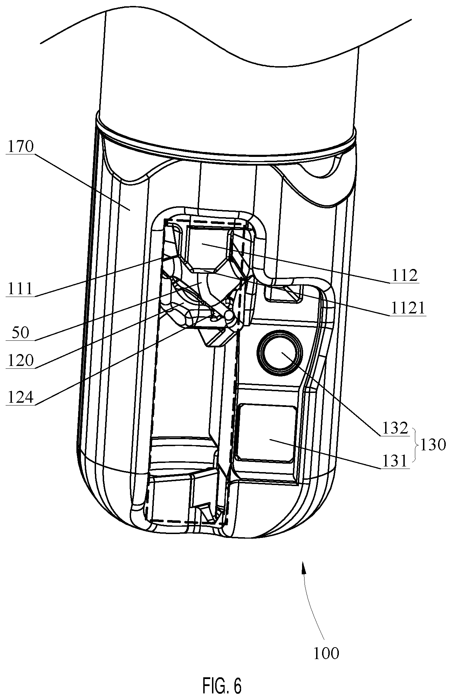

As shown in Figs. 2 to 9, the distal end portion 100 may include a distal end base 110, a raising base 120 and a distal end cap 170. The distal end base 110 may protect internal components of the distal end portion 100. Specifically, as shown in Fig. 6, the distal end cap 170 may surround the distal end base 110 to prevent the distal end base 110 from damaging the examined site.

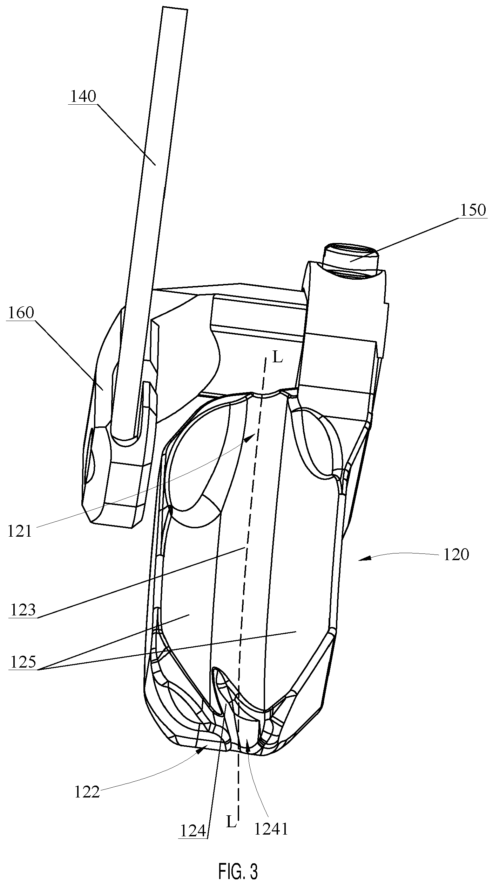

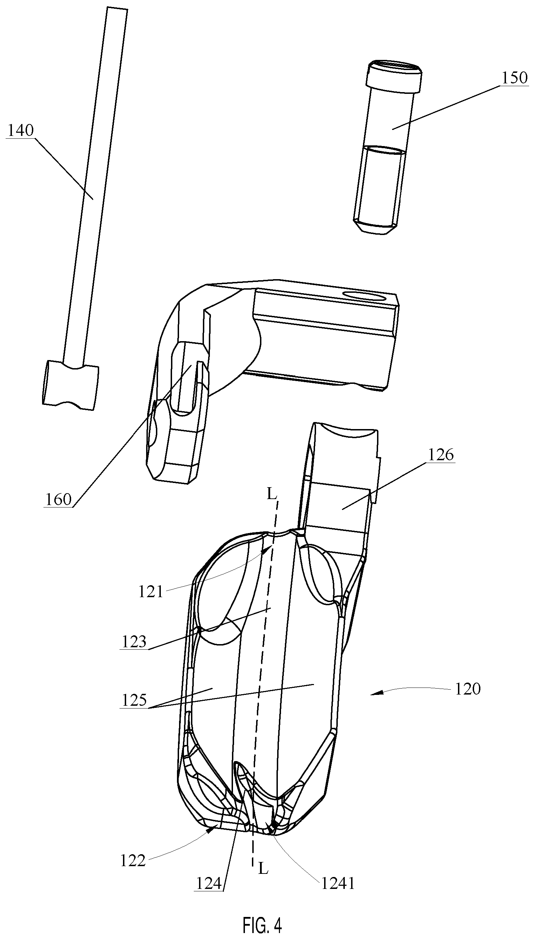

An accommodation chamber communicating with the instrument channel outlet 113 may be defined in the distal end base 110. An exit window 111 is defined on at least one side of the accommodation chamber. The raising base 120 is rotatably disposed in the accommodation chamber, and the raising base 120 may be exposed from the accommodation chamber through the exit window 111. The raising base 120 may be configured to be rotatable around a rotating portion 126 to adjust a direction of the medical instrument protruding from the exit window 111. The rotating portion 126 may be disposed in the accommodation chamber. Specifically, as shown in Figs. 3 and 4, the raising base 120 may be connected to a rotating arm 160 through a pin 150, and the raising base 120 may be rotatable around the rotating portion 126 by driving the rotating arm 160. A steel wire 140 may pass through the insertion unit 10, such that the steel wire 140 may have an operation end located at the operation unit 20 and a connection end located at the distal end portion 100. The connection end may be connected to the rotating arm 160. An operator can control the operation end at the operation unit 20, so as to drive the raising base 120 to rotate around the rotating portion 126. When a medical instrument is supported on the raising base 120, an angle at which the medical instrument protrudes from the distal end portion 100 can be changed accordingly to meet the requirements of diagnosis or treatment in different situations.

The raising base 120 may include a rotating shaft end 121 and a distal end 122. The distal end 122 of the raising base 120 is closer to the distal end of the distal end portion 100, and the rotating shaft end 121 of the raising base 120 is closer to the proximal end of the distal end portion 100. The rotating shaft end 121 of the raising base 120 may be located near the rotating portion 126. The raising base 120 may include a first guide surface 123 extending from the distal end 122 toward the rotating shaft end 121. The first guide surface 123 may be configured to support the medical instrument and guide the medical instrument along a predetermined extension path or a predetermined retraction path. The first guide surface 123 may be formed by a curved surface with an arc-shaped cross section, or may be formed by a V-shaped groove comprising two planar surfaces and an arc surface connecting therebetween, or may also be formed by any other structure functioning as a guide. The predetermined extension path refers to a path along which the medical instrument moves when the medical instrument is being advanced over the first guide surface 123. The predetermined retraction path refers to a path along which the medical instrument moves when the medical instrument is being retracted. The predetermined extension path or the predetermined retraction path may differ according to a specific application scenario. For example, for a duodenal endoscope, the predetermined extension path may be an arc with a certain curvature. A positioning groove 124 may also be formed at the distal end 122 of the raising base 120. Similar to the first guide surface 123, the positioning groove 124 may adopt any suitable structure with a guiding function. The positioning groove 124 is described in detail hereinafter in conjunction with specific embodiments. At least a portion of the positioning groove 124 may be formed in the first guide surface 123, such that the positioning groove 124 may be located in the extension path or retraction path. When the medical instrument is advanced along the predetermined extension path under the guidance of the first guide surface 123, the medical instrument can move from the first guide surface 123 to the positioning groove 124. The positioning groove 124 maintains the medical instrument located along the extension path or the retraction path in position. Optionally, the positioning groove 124 may be configured to maintain the medical instrument in position by exerting a clamping force to the medical instrument. Optionally, the positioning groove 124 may be configured to extend along an arc, thereby also maintaining the medical instrument in position. Such a positioning groove 124 can force a portion of the medical instrument in the positioning groove 124 to deform. Since the medical instrument generally has a certain ability to maintain its original state, this can increase the acting force between the contact surfaces of the medical instrument and the positioning groove 124. It should be appreciated that a large acting force may be generated between the contact surfaces of the positioning groove 124 and the medical instrument held by the positioning groove 124, such that the friction force between the contact surfaces of the positioning groove 124 and the medical instrument is increased. The retraction of the medical instrument along the predetermined retraction path is similar to the advancement of the medical instrument along the predetermined extension path, and is not repeated herein.

It can be understood that the medical instrument may include a first-specification instrument 50 of a smaller diameter and a second-specification instrument of a larger diameter. Exemplarily, the first-specification instrument 50 may be a guide wire, and the second-specification instrument may be a cannula or a papillotome. Since the first-specification instrument has a smaller diameter, a greater portion of the first-specification instrument can be received in the positioning groove 124, such that the retaining force exerted by the positioning groove 124 on the first-specification instrument is greater than that on the second-specification instrument. However, since the second-specification instrument generally has greater rigidity than the first-specification instrument, even if the retaining force exerted by the positioning groove 124 on the second-specification instrument is smaller, the second-specification instrument remains stable under the positioning of the positioning groove 124.

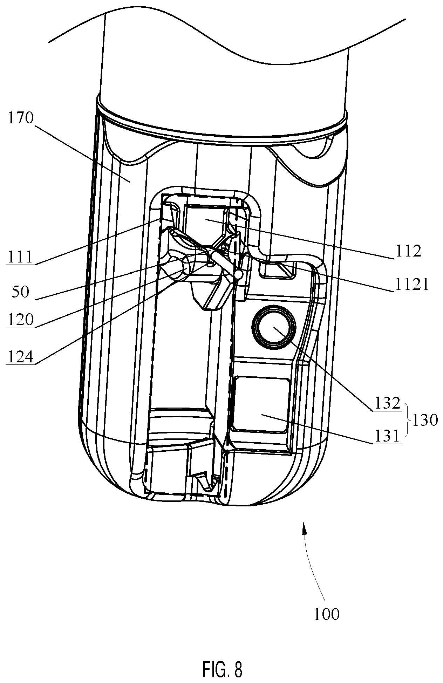

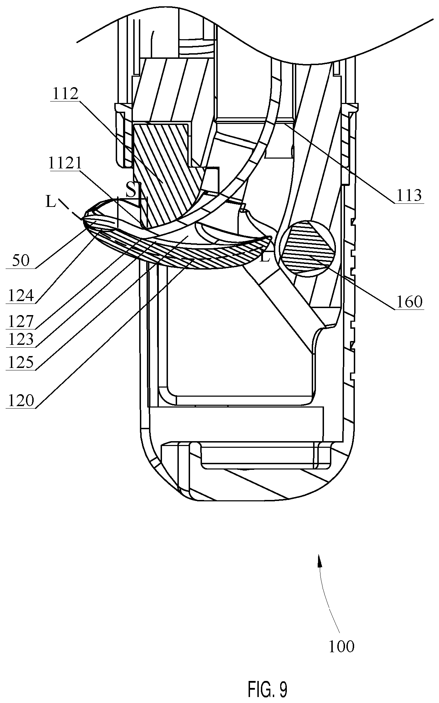

Further, as shown in Figs. 8 and 9, an abutment portion 112 may be provided at an edge of the exit window 111. The first guide surface 123 may include an abutment position 127 corresponding to the abutment portion 112. When the raising base 120 is lifted to a predetermined angle, the abutment position 127 cooperates with the abutment portion 112 to clamp the medical instrument supported on the first guide surface 123. Specifically, as shown in Fig. 9, the abutment position 127 may be located outside the positioning groove 124, and the abutment position 127 may be closer to the rotating shaft end 121 than the positioning groove 124 along the first guide surface 123. If no medical instrument is supported on the raising base 120, when the raising base 120 is lifted, the first guide surface 123 may abut against the abutment portion 112, or be spaced apart from the abutment portion 112 by a gap. The gap is smaller than the diameter of a first-specification instrument, such as a guide wire. When a medical instrument is supported on the raising base 120, the raising base 120 is lifted until the medical instrument is clamped by the first guide surface 123 and the abutment portion 112, such that the medical instrument is mechanically fixed between the abutment position 127 on the first guide surface 123 and the abutment portion 112.

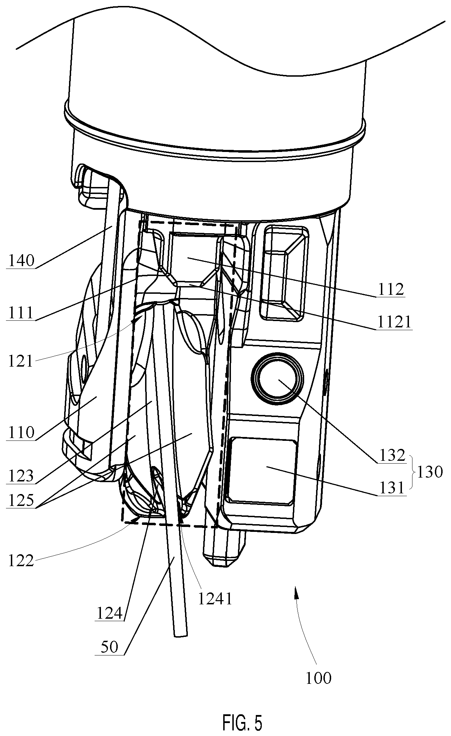

The raising base 120 assumes different states depending on its lifting angle. As shown in Fig. 5, the raising base 120 is in an initial state in which the raising base 120 has not been lifted. As shown in Figs. 6 and 7, the raising base 120 is in a semi-locking state in which the raising base 120 is lifted by a certain angle but does not reach the predetermined angle, and the medical instrument is constrained between the positioning groove 124 and the abutment portion 112. Since the raising base 120 is lifted by a certain angle which cause the medical instrument to deform to some extent, the friction force between the contact surfaces of the positioning groove 124 and the medical instrument is greater than that when the raising base 120 is in the initial state. It can be understood, when the raising base 120 is in the semi-locking state, the medical instrument is not mechanically clamped between the first guide surface 123 and the abutment portion 112, but the medical instrument may contact with the abutment portion 112. In this state, the medical instrument is partially deformed under the action of the abutment portion 112, but the force applied by the abutment portion 112 on the medical instrument has not reached a maximum value and is insufficient to securely lock the medical instrument. When the raising base 120 is in the semi-locking state, a retaining force on the medical instrument still mainly comes from the friction force between the contact surfaces of the positioning groove 124 and the medical instrument. As shown in Figs. 8 and 9, when the raising base 120 is lifted to the predetermined angle, the raising base 120 is fully lifted to a mechanical locking state. In this state, the medical instrument supported on the raising base 120 is clamped between the abutment position 127 of the first guide surface 123 and the abutment portion 112. The abutment position 127 on the first guide surface 123 is spaced from the positioning groove 124 by a spacing S. When the raising base 120 is in the mechanical locking state, the abutment portion 112 may securely lock the medical instrument together with the abutment position 127 of the first guide surface 123. In this state, the retaining forces on the medical instrument include both the friction force between the contact surfaces of the positioning groove 124 and the medical instrument, and the clamping force exerted by the abutment portion 112 and the first guide surface 123 on the medical instrument. It should be appreciated that in other embodiments, the spacing S may also be 0, i.e., the abutment position 127 may be located adjacent to the positioning groove 124 while remaining outside the positioning groove 124.

The endoscope of the present disclosure is described below with reference to the accompanying drawings, taking endoscopic retrograde cholangiopancreatography as an example.

First, a cannula is inserted through the instrument channel. A distal end of the cannula protrudes from the distal end portion 100 and is supported by the raising base 120. The raising base 120 is lifted to adjust a direction in which the cannula protrudes from the exit window 111, such that the cannula can reach an appropriate position in the subject. Then, a guide wire is inserted into a lumen of the cannula and advanced to the appropriate position in the subject along the lumen of the cannula. Subsequently, the cannula may be withdrawn. When a tip of the distal end of the cannula is retracted onto the raising base 120, the guide wire in the lumen of the cannula falls into the positioning groove 124. The guide wire is retained by the positioning groove 124, and specifically, the guide wire can be held in position due to friction between the contact surfaces of the positioning groove 124 and the guide wire. When the tip of the distal end of the cannula is further retracted into the instrument channel outlet 113, the guide wire falls onto the first guide surface 123. At this time, the raising base 120 is lifted by the predetermined angle, such that the abutment portion 112 cooperates with the first guide surface 123 to clamp the guide wire in a mechanically locked state. The cannula continues to be withdrawn. During retraction of the cannula from the positioning groove of the raising base to the instrument channel inlet, although the guide wire tends to retract due to friction between the cannula and the guide wire, the guide wire can stably maintain its position under the positioning action of the positioning groove 124 on the guide wire and/or the locking action formed by the abutment portion 112 clamping the guide wire in cooperation with the first guide surface 123. After the cannula is fully withdrawn, a papillotome may be advanced along the guide wire. When a distal tip of the papillotome reaches the instrument channel outlet 113 and encounters resistance, the lifting angle of the raising base 120 may be reduced to place the raising base 120 in a semi-locking state. The gap between the first guide surface 123 and the abutment portion 112 increases, providing space for further advancement of the papillotome. When the raising base 120 is in the semi-locking state and a portion of the guide wire exposed beyond the papillotome is received in the positioning groove 124, the positioning groove 124 still exerts a retaining force on the guide wire such that the position of the guide wire remains unchanged. Finally, the papillotome is further advanced along the guide wire to the appropriate position in the subject for diagnosis or treatment.

It should be appreciated that the above description is provided by way of example only, with reference to endoscopic retrograde cholangiopancreatography. The medical instrument may take any suitable form, and the use of the distal end portion of the endoscope of the present disclosure is not specifically limited herein.

In the distal end portion of the present disclosure, the medical instrument can be mechanically locked by being clamped between the abutment portion 112 and the first guide surface 123, and is thereby maintained in position. The positioning groove 124 may be configured to maintain the medical instrument located in the extension path or the retraction path in position. When the medical instrument is received in the positioning groove, relatively large friction can exist between the medical instrument and the positioning groove 124, such that the medical instrument is resistant to left-right shaking and/or forward-backward movement. Specifically, during replacement of the medical instrument under the guidance of the guide wire, for example, during retraction of the cannula along the guide wire in the instrument channel and during advancement of the papillotome along the guide wire in the instrument channel, the abutment portion 112 and the first guide surface 123 can clamp the guide wire, thereby mechanically locking the guide wire in position. However, during the process in which the distal tip of the cannula is retracted from the positioning groove 124 to the abutment position 127 (i.e., the mechanical locking position of the guide wire), or the process in which the distal tip of the papillotome is advanced from the abutment position 127 (i.e., the mechanical locking position of the guide wire) to the positioning groove 124, although the cannula or the papillotome is sleeved over the guide wire so that the abutment portion 112 cannot, together with the first guide surface 123, mechanically lock the guide wire, the portion of the guide wire exposed beyond the cannula or the papillotome is received in the positioning groove 124, so that the positioning groove 124 can form a radial constraint on the guide wire, thereby preventing lateral shifting of the guide wire. In addition, relatively large friction between the positioning groove 124 and the guide wire resists axial shifting of the guide wire. That is, when the distal tip of the medical instrument, such as a cannula or a papillotome, guided by the guide wire moves between the positioning groove 124 and the abutment position 127, the position of the guide wire can be maintained by the friction between the positioning groove 124 and the guide wire. When the distal tip of the medical instrument, such as a cannula or a papillotome, guided by the guide wire moves between the abutment position 127 and the instrument channel inlet, the guide wire can be maintained not only by friction between the positioning groove and the guide wire, but also by mechanical clamping of the abutment portion 112 and the first guide surface 123 on the guide wire. Thus, the raising base 120 of the present disclosure has a mechanical locking state and a semi-locking state. When replacing the medical instrument under the guidance of the guide wire, the position of the guide wire can be stably maintained, whether during replacement of the medical instrument on the raising base 120 or during movement of the medical instrument in the instrument channel. An operator can use such an endoscope more simply and conveniently. In addition, in the present disclosure, since the abutment position 127 for mechanically locking the medical instrument is on the first guide surface 123 and outside the positioning groove 124, the surface where the abutment position 127 is located has a relatively regular shape. As a result, the force on the guide wire can be more uniform, stress concentration can be reduced, and the medical instrument is less likely to be damaged. This avoids replacement of the medical instrument due to damage during use of the endoscope, thereby improving stability of the endoscope during use and enhancing the operator's use experience.

In an embodiment of the present disclosure, as shown in Figs. 2 to 5, two second guide surfaces 125 may be provided on the raising base 120, and the first guide surface 123 may be formed between the two second guide surfaces 125. The two second guide surfaces 125 may be configured to guide the medical instrument onto the first guide surface 123, such that the medical instrument tends to remain on the first guide surface 123 during retraction or advancement. That is, if the medical instrument deviates from the first guide surface 123 during advancement or retraction, once the external force is removed, the medical instrument returns to the first guide surface 123 under the guidance of the second guide surface 125, and moves along the predetermined extension path or the predetermined retraction path under the guidance of the first guide surface 123.

In an embodiment of the present disclosure, as shown in Figs. 5 to 9, the abutment portion 112 may include an abutment surface 1121. The abutment surface 1121 may be an arc-shaped surface. The abutment surface 1121 of the abutment portion 112 may cooperate with the abutment position 127 to clamp the medical instrument supported on the first guide surface 123. By contacting the medical instrument through the arc-shaped abutment surface 1121, the abutment portion 112 reduces stress concentration, thereby preventing damage to the medical instrument when the abutment portion 112 abuts against the medical instrument.

Exemplarily, when the raising base 120 is lifted by the aforementioned predetermined angle, the projection of the abutment surface 1121 on the raising base 120 may be covered by the first guide surface 123. Exemplarily, in the embodiment where the raising base 120 includes two second guide surfaces 125, when the raising base 120 is lifted by the predetermined angle, the abutment portion 112 abuts against the medical instrument without contacting the second guide surfaces 125.

In an embodiment of the present disclosure, as shown in Figs. 2 to 5, a positioning groove outlet 1241 may be defined at the distal end 122 of the raising base 120. The positioning groove 124 may be formed by extending from the positioning groove outlet 1241 toward the rotating shaft end 121 along a predetermined route. The medical instrument may include a first-specification instrument 50. The first-specification instrument 50 has a diameter less than or equal to a predetermined value such that in a cross section of the positioning groove outlet 1241, at least half of the first-specification instrument 50 may be received in the positioning groove outlet 1241. The positioning groove outlet 1241 is an outlet through which the medical instrument (particularly the first-specification instrument 50) protrudes out of the raising base 120. When, in a cross section of the positioning groove outlet 1241, at least half of the first-specification instrument 50 is received in the positioning groove outlet 1241, the positioning groove outlet 1241 may provide radial constraint on the first-specification instrument 50, thereby preventing radial shaking of the first-specification instrument 50 and improving stability of the guide wire.

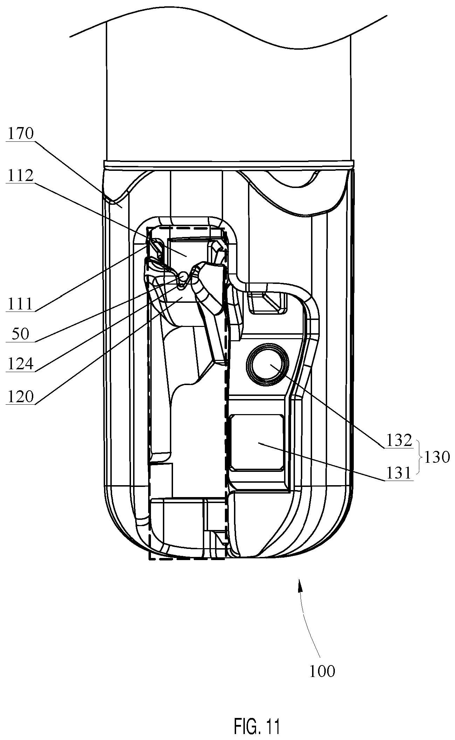

Exemplarily, when the raising base 120 is lifted to the predetermined angle, referring to Figs. 9 to 12 in combination, the abutment position 127 cooperates with the abutment portion 112 to clamp the first-specification instrument 50 supported on the first guide surface 123, and, in a cross section of the positioning groove outlet 1241, at least half of the first-specification instrument 50 may be received in the positioning groove outlet 1241. The raising base 120 is in the mechanical locking state with at least half of the first-specification instrument 50, in a cross section of the positioning groove outlet 1241, received in the positioning groove outlet 1241. A large friction exists between the first-specification instrument 50 and the positioning groove 124, which reduces the force required for the abutment portion 112 and the first guide surface 123 to clamp the first-specification instrument 50. Moreover, after the lifting angle of the raising base 120 is reduced to create a passage through which the medical instrument to be replaced can pass, the first-specification instrument 50, such as a guide wire, remains positioned in the positioning groove outlet 1241 without becoming unstable.

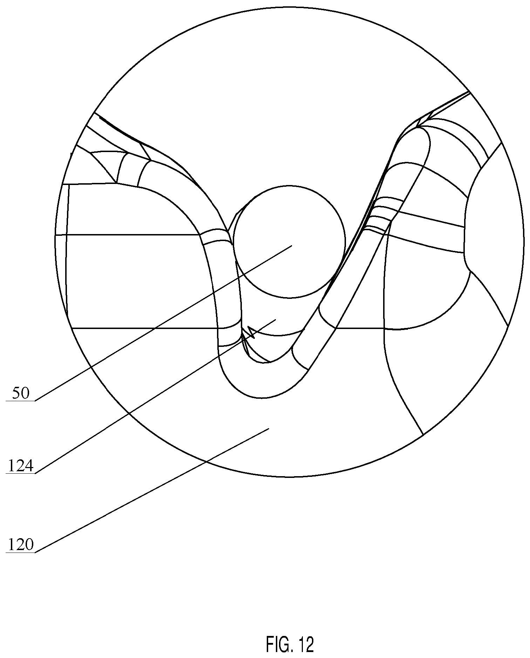

Exemplarily, the positioning groove outlet 1241 may be an opening of any suitable shape. Exemplarily, referring specifically to Fig. 12, on a cross section of the positioning groove outlet 1241 perpendicular to the aforementioned predetermined route, the positioning groove outlet 1241 may be generally V-shaped. It should be appreciated that the "V-shape" mentioned herein only describes the general shape of the cross section of the positioning groove outlet 1241, and does not strictly limit the cross section of the positioning groove outlet 1241 to a "V" shape. For example, the cross section of the positioning groove outlet 1241 may be a V-shape with arc-shaped side edges, or a V-shape with rounded bottom, or any combination of multiple shapes. Such a positioning groove outlet 1241 can provide radial constraint for the medical instrument, for example, prevent the medical instrument from shifting in a direction perpendicular to the extension path, and accommodate medical instruments of various sizes. For a first-specification instrument 50 of a smaller diameter, it can be received in a position of the positioning groove outlet 1241 corresponding to its diameter. For a second-specification instrument of a larger diameter, the V-shaped opening edges of the positioning groove outlet 1241 can support the second-specification instrument and provide certain degree of radial constraint thereto.

Exemplarily, as shown in Fig. 4, the first guide surface 123 may extend along a longitudinal axis L-L of the raising base 120, and the predetermined route may intersect with the longitudinal axis L-L of the raising base 120. The predetermined route may take any suitable shape, such as an arc, a curve, a polyline. The predetermined route is used herein to define a course of the positioning groove 124 without limiting variations in a size of the positioning groove 124 in a direction perpendicular to the predetermined route along the predetermined route. The predetermined route may be inclined to the longitudinal axis L-L of the raising base 120, such that a distance between the predetermined route and the longitudinal axis L-L of the raising base 120 may first decrease and then increase along a direction from the distal end 122 to the rotating shaft end 121. The predetermined route may intersect with the longitudinal axis L-L of the raising base 120. Such a positioning groove 124 can force the medical instrument received therein to deform to a greater extent, thereby further increasing friction between the contact surfaces of the medical instrument and the positioning groove 124 and enhancing the positioning effect of the positioning groove 124 on the medical instrument. When at least a portion of the medical instrument is received in the positioning groove 124, the medical instrument can maintain its position more stably.

As mentioned above, the distal end portion 100 may include an optical imaging assembly 130. The optical imaging assembly 130 may have an imaging region, and the optical imaging assembly 130 may generate an optical image of a subject within the imaging region. Exemplarily, the optical imaging assembly 130 and the exit window 111 may be located on the same side of the distal end portion 100. The predetermined route may be inclined toward a center of the imaging region of the optical imaging assembly 130. By the positioning of the positioning groove 124, the portion of the medical instrument protruding from the distal end of the raising base 120 may enter the imaging region of the optical imaging assembly 130. In this way, a blind spot in the visual field can be reduced during the operation of the medical instrument. When an operator operates the medical instrument to control the portion of the medical instrument protruding from the exit window 111 so as to assist in diagnosis or treatment, real-time imaging of the optical imaging assembly 130 may be utilized to observe the position of the distal end of the medical instrument. In this way, the effect of diagnosis or treatment can be improved.

Exemplarily, the opening of the positioning groove 124 is tapered from the distal end 122 toward the rotating shaft end 121 along the predetermined route. A projection of the positioning groove 124 on the first guide surface 123 is generally V-shaped. Such a positioning groove 124 facilitates smooth movement of the medical instrument between the first guide surface 123 and the positioning groove 124, and accommodates medical instruments of various sizes, thereby providing a broader application scope of the endoscope.

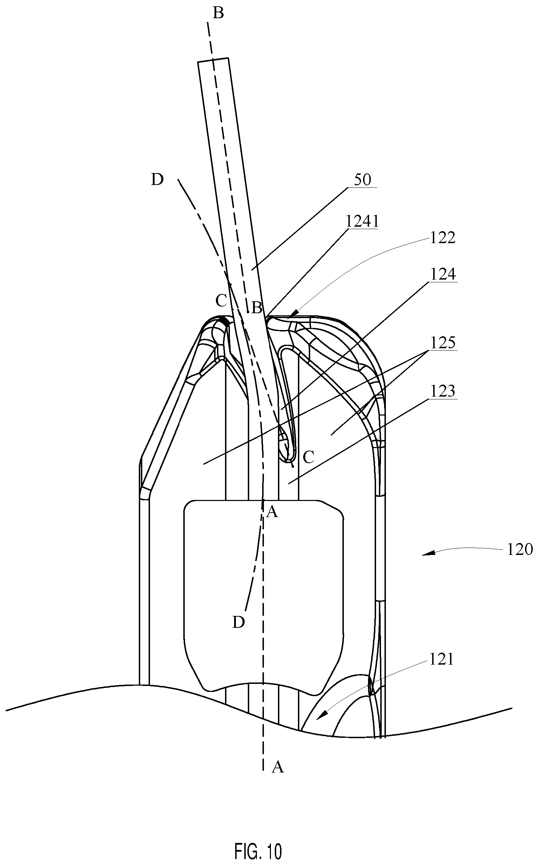

Exemplarily, as shown in Figs. 8 and 9, the first guide surface 123 may extend along the longitudinal axis L-L of the raising base 120. The abutment position 127 is spaced from the positioning groove 124 by a spacing S. As shown in Fig. 10, in an embodiment where a course of the positioning groove 124 is inclined relative to a course of the first guide surface 123, the medical instrument is bent when advanced from the first guide surface 123 to the positioning groove 124. The greater the inclination of the course of the positioning groove 124 relative to the course of the first guide surface 123, the greater the deformation of the medical instrument. If the course of the positioning groove 124 is excessively inclined relative to the course of the first guide surface 123, the medical instrument may escape from the positioning groove 124 due to a large reaction force. During advancement of the medical instrument along the predetermined extension path under the guidance of the first guide surface 123, the medical instrument extends approximately along the longitudinal axis L-L of the raising base 120. In this embodiment, after passing through the abutment position 127, the medical instrument travels across a non-zero spacing S before reaching the positioning groove 124, which provides a buffer space for directional adjustment of the medical instrument. That is, a portion of the medical instrument located within the spacing S can also deform to compensate for the deviation between the course of the positioning groove 124 and the course of the first guide surface 123, thereby avoiding excessive local bending of the medical instrument. In this way, the medical instrument is prevented from escaping from the positioning groove 124 due to excessive deformation while being in the mechanically locked state, which results in deviation from the predetermined extension path or retraction path. With such an endoscope, the distal end of the medical instrument can be advanced to the predetermined position more reliably, and the diagnostic or therapeutic efficacy of the endoscope is improved.

In an embodiment of the present disclosure, as shown in Fig. 10, the extension path may include a first extension sub-path (indicated by A-A) and a second extension sub-path (indicated by B-B). The first extension sub-path A-A generally corresponds to a path of the first-specification instrument 50 on the first guide surface 123. The second extension sub-path B-B generally corresponds to a path of the first-specification instrument 50 beyond the distal end of the raising base 120. Similarly, the retraction path may include a first retraction sub-path and a second retraction sub-path. The first guide surface 123 may be used to guide the first-specification instrument 50 to the first extension sub-path A-A or the first retraction sub-path. The positioning groove 124 may be used to guide the first-specification instrument 50 located in the first extension sub-path A-A to the second extension sub-path B-B, or guide the first-specification instrument 50 located in the second retraction sub-path to the first retraction sub-path. The positioning groove 124 can not only hold the first-specification instrument 50, but also guide the first-specification instrument 50. Taking advancement along the extension path as an example, when the first-specification instrument 50 travels along the first extension sub-path A-A toward the distal end of the raising base 120 until it just contacts the positioning groove 124, the distal end of the first-specification instrument 50 changes direction mainly under the guidance of the right side wall of the positioning groove 124, while the left side wall of the positioning groove 124 can maintain the position of the first-specification instrument 50 along the lateral direction. In particular, for the first-specification instrument 50 of a smaller diameter, at the positioning groove outlet 1241 of the positioning groove 124, the first-specification instrument 50 may only contact and abut against the right side wall of the positioning groove 124. Therefore, the predetermined route along which the positioning groove 124 extends (indicated by C-C) may not be completely collinear with the second extension sub-path B-B. If a medical instrument contacts and abuts against both the left and right side walls of the positioning groove 124 at the positioning groove outlet 1241, the predetermined route along which the positioning groove 124 extends (indicated by line C-C) may be generally collinear with the second extension sub-path B-B. With an appropriate configuration of the positioning groove 124, the positioning groove 124 can not only force the first-specification instrument 50 received therein to deform, thereby increasing friction between the contact surfaces of the positioning groove 124 and the first-specification instrument 50, but also enable the distal end of the first-specification instrument 50 to protrude from the distal end of the raising base 120 along an desired direction (for example, along sub-path B-B) after deformation. When such a distal end portion is equipped with the first-specification instrument 50, the distal end of the first-specification instrument 50 can be positioned more accurately in the subject. The retraction path is similar to the extension path and is not repeated herein. Exemplarily, as shown in Fig. 10, the predetermined route C-C and a central axis of the first guide surface 123 (i.e., the first extension sub-path A-A or the first retraction sub-path) jointly define an inscribed circle passing through the positioning groove outlet 1241 and the abutment position 127 (the illustrated arc D-D is a part of the inscribed circle). A diameter of the inscribed circle is greater than a minimum bending diameter of the first-specification instrument 50. The minimum bending diameter of the first-specification instrument 50 refers to a minimum diameter to which the first-specification instrument 50 can bend within an elastic range. If the first-specification instrument 50 is bent to a diameter smaller than the minimum bending diameter, the first-specification instrument 50 may undergo irreversible deformation. When such a distal end portion is equipped with the first-specification instrument 50, the first-specification instrument 50 is elastically bent under the positioning of the positioning groove 124. In this manner, instability or irreversible bending of the first-specification instrument 50 can be prevented, thereby avoiding replacement of the first-specification instrument 50 due to damage during use and also preventing the first-specification instrument 50 from escaping from the positioning groove 124 due to excessive deformation. Therefore, the user experience of the operator is improved.

In the description of the present disclosure, it is to be understood that orientation or positional relationships indicated by orientation words, such as "front", "rear", "upper", "lower", "left", "right", "lateral direction", "perpendicular direction", "perpendicular", "horizontal", "top", "bottom", usually are shown based on the accompanying drawings, only for the purposes of the ease in describing the present disclosure and simplification of its descriptions. Unless stated to the contrary, these orientation words do not indicate or imply that the specified apparatus or element has to be specifically located, and structured and operated in a specific direction, and therefore, should not be understood as limitations to the present disclosure. The orientation words "inside" and "outside" refer to the inside and outside relative to the contour of each component itself.

For facilitating description, the spatial relative terms such as "on", "above", "on an upper surface of " and "upper" may be used here to describe a spatial position relationship between one or more components or features and other components or features shown in the accompanying drawings. It should be understood that the spatial relative terms not only include the orientations of the components shown in the accompanying drawings, but also include different orientations in use or operation. For example, if the component in the accompanying drawings is turned upside down completely, the component "above other components or features" or "on other components or features" will include the case where the component is "below other components or features" or "under other components or features". Thus, the exemplary term "above" can encompass both the orientations of "above" and "below". In addition, these components or features may be otherwise oriented (for example rotated by 90 degrees or other angles) and the present disclosure is intended to include all these cases.

It should be appreciated that the terms used herein are only for describing specific embodiments, and are not intended to limit the exemplary embodiments according to the present application. As used herein, an expression of a singular form includes an expression of a plural form unless otherwise indicated. In addition, it should also be understood that when the terms "including" and/or "comprising" are used herein, it indicates the presence of features, steps, operations, parts, components and/or combinations thereof.

It should be appreciated that the terms "first", "second" and the like in the description and claims, as well as the above accompanying drawings, of the present disclosure are used to distinguish similar objects, but not necessarily used to describe a specific order or precedence order. It should be understood that ordinal numbers used in this way can be interchanged as appropriate, so that the embodiments of the present disclosure described herein can be implemented in a sequence other than those illustrated or described herein.

The present disclosure has been illustrated by the above several embodiments, but it should be understood that the above embodiments are only for the purpose of illustration and description, and are not intended to limit the present disclosure to the scope of the described embodiments. Moreover, those skilled in the art can make more variations, modifications and improvements according to the teachings of the present disclosure, and these variations, modifications and improvements all fall within the spirit and the scope of protection of the present disclosure. The scope of protection of the present disclosure is defined by the appended claims and their equivalent scope.

## Claims

1. A distal end portion of an endoscope, comprising:
a distal end base having an accommodation chamber defined therein, the accommodation chamber communicating with an instrument channel of the endoscope and having an exit window for a medical instrument to protrude therethrough, and an abutment portion being provided at an edge of the exit window; and
a raising base rotatably disposed in the accommodation chamber and configured to adjust a direction in which the medical instrument protrudes from the exit window, the raising base comprising a rotating shaft end and a distal end, wherein:
the raising base comprises a first guide surface extending from the distal end toward the rotating shaft end, the first guide surface is configured to support the medical instrument and guide the medical instrument along a predetermined extension path or a predetermined retraction path, the first guide surface comprises an abutment position corresponding to the abutment portion, the abutment position is configured to cooperate with the abutment portion to clamp the medical instrument supported on the first guide surface when the raising base is lifted to a predetermined angle;
the raising base further comprises a positioning groove at the distal end, at least a portion of the positioning groove is formed in the first guide surface, the positioning groove is configured to maintain the medical instrument in the extension path or the retraction path in position; and
the abutment position is located outside the positioning groove.

2. The distal end portion according to Claim 1, wherein a positioning groove outlet is defined at the distal end of the raising base, and the positioning groove is formed by extending from the positioning groove outlet toward the rotating shaft end along a predetermined route;
the medical instrument comprises a first-specification instrument which has a diameter less than or equal to a predetermined value such that at least half of the first-specification instrument is receivable in the positioning groove outlet.

3. The distal end portion according to Claim 2, wherein when an instrument supported on the first guide surface is the first-specification instrument and the raising base is lifted to the predetermined angle, at least half of the first-specification instrument is received in the positioning groove outlet.

4. The distal end portion according to Claim 2 or 3, wherein a cross section of the positioning groove outlet is V-shaped, and the cross section is perpendicular to the predetermined route.

5. The distal end portion according to any one of Claims 2-4, wherein an opening of the positioning groove is tapered from the distal end toward the rotating shaft end along the predetermined route.

6. The distal end portion according to any one of Claims 2-5, wherein the first guide surface extends along a longitudinal axis of the raising base, and the predetermined route intersects with the longitudinal axis of the raising base.

7. The distal end portion according to Claim 6, wherein the abutment position is spaced apart from the positioning groove with a non-zero spacing.

8. The distal end portion according to Claim 6 or 7, wherein a circle internally tangent to both the predetermined route and a central axis of the first guide surface, and passing through the positioning groove outlet and the abutment position, has a diameter greater than a minimum bending diameter of the first-specification instrument.

9. The distal end portion according to any one of Claims 6-8, further comprising an optical imaging assembly located on a same side of the distal end base as the exit window, wherein the predetermined route is inclined toward a center of an imaging region of the optical imaging assembly.

10. An endoscope, comprising the distal end portion according to any one of Claims 1 to 9.
